(19) Europäisches Patentamt
European Patent Office
Office européen des brevets

(11) Publication number: **0 111 605**
**B1**

(12) **EUROPEAN PATENT SPECIFICATION**

(45) Date of publication of patent specification: **09.04.86**

(51) Int. Cl.$^4$: **C 07 C 51/353,** C 07 C 57/04, C 07 C 69/54, C 07 C 67/343

(21) Application number: **82306820.0**

(22) Date of filing: **21.12.82**

(54) **Preparation of unsaturated acids and esters by oxidative condensation.**

(43) Date of publication of application:
**27.06.84 Bulletin 84/26**

(45) Publication of the grant of the patent:
**09.04.86 Bulletin 86/15**

(84) Designated Contracting States:
**BE CH DE FR GB IT LI LU NL SE**

(56) References cited:
**EP-A-0 033 881**
**DE-A-1 294 956**
**GB-A-1 207 415**
**GB-A-1 207 416**

(73) Proprietor: **THE STANDARD OIL COMPANY**
**Midland Building**
**Cleveland, Ohio 44115 (US)**

(72) Inventor: **Grasselli, Robert Karl**
**150 Greentree Road**
**Chagrin Falls Ohio 44022 (US)**
Inventor: **Guttmann, Andrew Tytus**
**5241 Philip Street**
**Maple Heights Ohio 44137 (US)**

(74) Representative: **Smith, Sydney et al**
**Elkington and Fife High Holborn House**
**52/54 High Holborn**
**London WC1V 6SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 111 605 B1

**Description**

This invention relates to a novel vapor phase process for the production of unsaturated acids and esters, such as acrylic acid and methyl acrylate.

Preparation of unsaturated acid derivatives from corresponding saturated compounds by condensation with formaldehyde is known. For example, U.S. Patent No. 3,701,798 discloses the production of unsaturated acid derivatives through condensation with formaldehyde over catalysts containing rare earth oxides. U.S. Patent No. 3,014,958 discloses an improvement for making unsaturated esters from formaldehyde and other esters wherein the feed contains more than 7% of the desired unsaturated ester. Catalysts used in this reference are phosphates and mixed oxides.

U.S. Patent No. 3,933,888, U.S. 3,578,702 and U.S. 4,165,438 all disclose reactions using formaldehyde over various oxide catalysts such as vanadium-phosphorus, metals of the lanthanide series, or metals found in Groups IIA and Group IIIA of the Periodic Table.

In DE—B—1,294,956 there is described a process for the preparation of methyl acrylate by oxidation in the vapor phase with oxygen of methyl acetate or a mixture of methyl acetate and methanol with subsequent condensation of the formaldehyde with methyl acetate wherein the conversion of the methyl acetate or mixture thereof with methanol takes place in a single stage at a temperature of 180 to 500°C in the presence of a catalyst which comprises 15 to 40 per cent by weight of titanium dioxide, 20 to 40 per cent by weight vanadium pentoxide and 20 to 65 per cent by weight of phosphoric acid.

In GB—A—1,207,416 a process is also described in which methyl acrylate is prepared by reaction of methanol and methyl acetate in the presence of oxygen. In this case the catalyst used is copper or silver carried on an aluminosilicate support which has been treated with a base before the silver or copper is deposited thereon.

In EP—A—3381 a process is described for the preparation of methyl methacrylate by reaction of methyl propionate and methanol in the presence of a catalyst mixture which acts to catalyse condensation and is also suitable for the dehydrogenation of methanol to formaldehyde. Catalysts useful in the process contain copper and zinc as well as tellurium and/or selenium and/or sulfur, if desired in oxidised form. Such catalysts are operative in the absence of gaseous oxygen, although traces of oxygen do not damage the process.

It has now been discovered that other catalysts may be used with advantage in the production of derivatives of unsaturated acids by the reaction of a saturated derivative with methanol and oxygen, over a suitable catalyst.

The invention provides therefore a process for the production of unsaturated acids and esters which comprises reacting in the vapor phase at a temperature of 200°C to 500°C a first reactant selected from saturated monocarboxylic aids, esters and anhydrides, a second reactant selected from primary and secondary alcohols and di-alkyl ethers, and oxygen, in the presence of an oxidation catalyst, said catalyst having the empirical formula:

$$A_aB_bC_cPO_x$$

where

A = alkali metal, alkaline earth metal, Tl, or mixtures thereof

B = Fe, Co, Ni, Cr, Mn, Cu, Ag, Pt, Pd, Rh, Ru, or mixtures thereof

C = Sc, Y, La, rare earth metals, Th, U, Nb, Si, Ge, Ta, Mo, W, Bi, Sb, Te or mixtures thereof

where

a = 0—1.5

b = 0.01—3.0

c = 0—2.0

x = the number of oxygens required to satisfy the valence requirements of the other elements present.

Preferably the values of a, b and c should be such that the value of $an_a + bn_b + cn_c$ is 1 to 5, where $n_a$ is the valency of the ion or ions from Group A, $n_b$ is the valency of the ion (or ions) from Group B, and $n_c$ is the valency of the ion or ions from Group C.

The catalysts are prepared by methods known in the art; some typical methods of preparation are shown in the examples.

In another aspect of the invention, catalysts containing antimony and vanadium have proved very useful. They have the general formula:

$$A_aB_bC_cVSb_mO_x$$

where

A = alkali metal, alkaline earth metal, Tl, La, rare earth metal, Th, or mixtures thereof;

B = Cu, Ag, Fe, Co, Ni, Mn, Cr, Nb, Ta, Ti, P, As, Sn, B, U, or mixtures thereof;

C = Mo, W, Te, Bi, or mixtures thereof;

a = 0—1;

b = 0—1;

c = 0—1;

m = 0.5—40, preferably 3—15;

x = the number of oxygens required to satisfy the valence requirements of the other elements present.

Preferably, the value of a + b + c should be equal to or smaller than 2 but less than m.

The catalysts containing V and Sb, as shown above, are conveniently prepared by a redox reaction such as

$$V_2O_5 + Sb_2O_3 \rightarrow 2\ VSbO_4$$

in which pentavalent vanadium is reduced, while the trivalent antimony of $Sb_2O_3$ is oxidized to the pentavalent state. The $V_2O_5$ can be replaced by ammonium metavanadate or other compounds of pentavalent vanadium. Where excess $Sb_2O_3$ used, such that m > 1, such excess will, during the calcination of the catalyst at a high temperature in air, be oxidized to $Sb_2O_4$, $Sb_6O_{13}$, and/or $Sb_2O_5$, or the like.

Alternatively, the excess antimony in compositions with m > 1 can be supplied as a compound of pentavalent antimony, eg. $Sb_2O_5$. In such a case the excess antimony is preferably added after the redox reaction shown above had been completed. Thus, the catalytic compositions contain all or part of the vanadium in an oxidation state of less than 5+, and most of the antimony in the oxidation states of 4+ and 5+. The preparative methods include, for example, heating an intimate mixture of the dry reactants or, preferably, using an aqueous slurry method as illustrated in the examples. Alternatively, the catalysts may be prepared by reducing the pentavalent vanadium (e.g. $V_2O_5$) separately followed by reaction with a compound of pentavalent antimony e.g. $SbCl_5$ or $Sb_2O_5$. In all methods, supports and additional promoting and modifying elements may be added at different stages of the preparation.

Mixtures of molybdates with the phosphates and VSb catalysts described above, may be used as catalysts.

The catalysts described herein may be used unsupported, but the use of a suitable carrier, such as silica, alumina, amorphous silica-alumina, mixtures of silica and alumina, crystalline aluminosilicates, titania, zirconia, $BPO_4$, $SbPO_4$ natural clays, and similar materials is often preferred. Good results have been obtained using alumina with the mixed phosphates, and mixtures of alumina and silica with the catalysts containing vanadium and antimony described above. The concentration of the active catalyst on the support may range from about 5 to about 95% by weight.

The first reactant feed for the process of the present invention may be a saturated monocarboxylic acid, ester, or anhydride having two to fourteen carbon atoms. It is preferred to utilize acids and esters. The preferred acids and esters are acetic acid and acetates, and propionic acid and propionates. While these compounds are preferred, esters of other aliphatic acids may also be used.

The alcohol in the second reactant is a primary or secondary alcohol of which ethanol, n-propanol, n-butanol, and isobutanol are preferred. The particularly preferred alcohol is methanol.

It may be advantageous to use an ether such as dimethyl ether as feed instead of methanol. Both compounds are easily and selectively interconvertible over a variety of dehydration/hydration catalysts, e.g. gamma-alumina. Thus, if desired, dimethyl ether or its mixtures with the methanol may serve as an alternate feed equivalent to methanol alone.

The oxygen necessary for the oxidative condensation may be supplied as oxygen or an oxygen-containing gas such as air.

The oxidative condensation is conveniently carried out by mixing the alcohol such as methanol, with the appropriate acid derivative such as methyl propionate, in the desired ratio, vaporizing the mixture, and passing the vapors along with oxygen over a suitable catalyst. If dimethyl ether is used as feed, it may be introduced together with the oxygen and any gaseous diluent, added dissolved in the liquid feed, or introduced both ways. Fixed or fluid bed operation is possible, and inert diluents such as steam or nitrogen may be added.

The reaction temperature may range from 200 to about 500°C, preferably from 300 to 450°C; the average residence time may be from 2 to about 60 seconds, preferably from 5 to 20 seconds. The molar ratio of the acid derivative to the alcohol may range from 1:10 to 10:1; the molar ratio of oxygen to the alcohol is suitably from 0.2:1 to 10:1.

The following Examples illustrate the invention:

## Example 1

A catalyst having the composition 50% $K_{0.4}Th_{0.48}$—$Cu_{0.48}POx$ + 50% $Al_2O_3$ was prepared as follows. A slurry of 44.1 g hydrated alumina (Catapal SB, 85% $Al_2O_3$) in 170 ml $H_2O$ was heated to 70°C and a solution of 19.4 g $(NH_4)_2HPO_4$ in 50 ml $H_2O$ was added with stirring, followed by the addition of a warm solution of 39.1 g $(Th(NO_3)_4.4H_2O$ and 17.1 g $Cu(NO_3)_2.3H_2O$ in 65 ml $H_2O$, and a solution of 5.9 g $KNO_3$ in 10 ml $H_2O$. The slurry was evaporated to a low volume at about 90°C then dried at 120—125°C. The dry material was treated 3 hours at 290°C, 3 hours at 350°C, ground and screened to 20/35 mesh size, and finally calcined 5 hours at 500°C.

## Examples 2—10

Following the procedure of Example 1, various promoted copper-phosphate catalysts were prepared as shown in Table 1.

The catalysts were charged to a fixed-bed micro-reactor equipped with a preheat leg, serving as a vaporizer, and immersed in a temperature-controlled salt bath at 330°C. Liquid feed consisting of a mixture

of methyl acetate and methanol in a molar ratio of 10:1 was injected by a syringe pump into the reactor, through the preheat leg over a period of 100 minutes. A mixture of 10 vol.% oxygen and 90 vol.% nitrogen was introduced into the reactor through the preheat leg at the same time. The respective flow rates of the liquid and the gaseous feed were such that a reactant ratio methyl acetate/methanol/$O_2$/$N_2$ = 10/1/0.8/7.2 was obtained, and the average residence time in the reactor was 10 seconds. The reactor effluent was condensed, weighed, and analyzed by gas chromatography. The results of these experiments are shown in Table 1.

Examples 11—16

In the same manner as Example 1, catalysts containing phosphorus promoted with iron, chromium, cobalt, silver and molybdenum were prepared and run as per Examples 2—10 at 330°C with a contact time of 10 seconds. The result of these experiments are shown in Table II.

Example 17

A catalyst having the composition 42% $VSbO_x$, 42% $Al_2O_3$, and 16% $SiO_2$ was prepared as follows. A slurry of 25.9 g $Sb_2O_3$ in 100 ml $H_2O$ was heated to 75°C, and a hot solution of 2.8 g $NH_4VO_3$ in 300 ml $H_2O$ was added with stirring. The mixture was boiled under reflux overnight. The resulting dark-green liquid was mixed with 49.4 g hydrated alumina, and with 40.0 g 40% silica sol. The mixture was stirred in an open beaker, evaporated at 85—90°C, and dried at 120—125°C. The dried material was treated 5 hours at 350°C, ground and screened to 20/35 mesh size, and calcined 5 hours at 550°C.

Examples 18—25

In the same manner as Example 17, catalysts containing vanadium-antimony and promoted by potassium, copper, silver, iron, molybdenum and uranium were prepared. Catalysts of these examples were calcined at 550°C, except for Example 19 which was calcined at 650°C.

The catalysts of Examples 17—25 were tested according to the procedure of Examples 2—10 at a temperature of 330°C and a contact time of 10 seconds. The results are shown in Table III.

Examples 26—29

In the manner previously disclosed, catalysts of the invention were prepared having the composition set forth in Table IV.

Examples 26 and 29 had a support of 50% $Al_2O_3$, while Examples 27 and 28 had a support of 42% $AlO_3$, 16% $SiO_2$.

A feed of methyl propionate, methanol and oxygen was passed over the catalyst at a ratio of methyl-propionate/methanol of 10/1, and a ratio of oxygen/methanol as shown in the Table. The reaction temperature was 330°C, with a contact time as shown. The results are presented in Table IV.

The following example illustrates the use of dimethyl ether as the second reactant in the oxidative condensation with methyl acetate.

Example 30

The reactor was charged with a catalyst having the composition 50% $K_{0.4}Ce_{0.58}Cu_{0.58}PO_x$ + 50% $Al_2O_3$ (see Example 7). The testing procedure was essentially the same as that of Examples 2—10, except that the liquid feed consisted of methyl acetate alone, while the gaseous feed consisted of oxygen, nitrogen, and dimethyl ether. The composition of the gaseous feed and the respective flow rates of the liquid and the gaseous feeds were such that a reactant ratio methyl acetate/dimethyl ether/$O_2$/$N_2$ = 10/0.5/0.8/7.2 was obtained, and the average residence time in the reactor was 10 seconds. At the reaction temperature of 330°C, 45.2% ppc of methyl acrylate and 4.6% ppc acrylic acid were obtained, resulting in 49.8% ppc to total product. At 350°C the yields were 82.1% ppc to methyl acrylate and 13.6% ppc to acrylic acid, resulting in 95.7% ppc to total product.

The following example illustrates the use of a molybdate as oxidation catalyst.

Example 31

A catalyst having the composition 50% $Fe_2MO_{5.4}O_x$ and 50% $Al_2O_3$ was prepared as follows. To a warm slurry of 59 g Catapal SB in 230 ml $H_2O$ there was added a solution of 50.9 g ammonium heptamolybdate in 90 ml $H_2O$, and a solution of 43.1 g $Fe(NO_3)_3.9H_2O$ in 30 ml $H_2O$. The slurry was stirred and evaporated at 90°C, dried at 125°C, treated 3 hours at 290°C, 3 hours at 350°C, ground and screened, then calcined 5 hours at 430°C. When tested according to Examples 2—10, with Me-acetate and methanol in the feed, 24.2% ppc to methyl acrylate and 12.4% ppc to acrylic acid were obtained, resulting in 36.6% ppc to total product.

The following example illustrates the use of a zeolite exchanged with alkali metal and other elements as oxidation catalyst.

Example 32

A sample of commercial Zeolite 13X (sodium form) was exchanged with cesium by conventional treatment with an excess of $Cs_2CO_3$ solution, washing and drying. The dried material was impregnated with Fe and Mo to a total of approximately 8 wt.% by treatment with aqueous solutions of ammonium heptamolybdate and ferric nitrate, evaporating, drying at 125°C and treated 3 hours at 290°C. This material (12.6 g) was then mixed with 10.5 g 40% silica sol (4.2 g $SiO_2$), dried, ground and screened, then calcined 5

hours at 450°C. This catalyst, when tested according to the procedure of Examples 2—10 gave a yield of 7.3% ppc to methyl acrylate, and 0.2% ppc to acrylic acid.

TABLE I
Reaction of Methyl Acetate with Methanol and Oxygen Over Mixed Phosphates of Copper

| | | Per Pass Conversion % | | |
|---|---|---|---|---|
| Example | Catalyst | Methyl Acrylate | Acrylic Acid | Total |
| 1 | $K_{0.4}Th_{0.48}Cu_{0.48}\ PO_x$ | 74.9 | 10.9 | 85.8 |
| 2 | $K_{0.4}Th_{0.6}Cu_{0.24}\ PO_x$ | 64.5 | 6.5 | 71.0 |
| 3 | $K_{0.4}Th_{0.48}Cu_{0.48}V_{0.1}\ PO_x$ | 61.7 | 4.1 | 65.8 |
| 4 | $K_{0.4}Th_{0.48}Cu_{0.48}Te_{0.1}\ PO_x$ | 10.4 | —0— | 10.4 |
| 5 | $K_{0.4}Cu_{1.45}\ PO_x$ | 22.5 | 1.5 | 24.0 |
| 6 | $K_{0.4}La_{0.58}Cu_{0.58}\ PO_x$ | 66.9 | 3.8 | 70.7 |
| 7 | $K_{0.4}Ce_{0.58}Cu_{0.58}\ PO_x$ | 89.9 | 12.9 | 102.8 |
| 8 | $K_{0.4}Cr_{0.58}Cu_{0.58}\ PO_x$ | 41.2 | 11.6 | 52.8 |
| 9 | $K_{0.4}U_{0.725}Cu_{0.725}\ PO_x$ | 27.0 | 4.3 | 31.3 |
| 10 | $K_{0.4}Bi_{0.58}Cu_{0.58}\ PO_x$ | 10.8 | 2.1 | 12.9 |

TABLE II
Reaction of Methyl Acetate with Methanol and Oxygen Over Promoted Phosphate Catalysts

| | | Per Pass Conversion % | | |
|---|---|---|---|---|
| Example | Catalyst | Methyl Acrylate | Acrylic Acid | Total |
| 11 | $K_{0.4}Fe_{0.57}\ PO_x$ | 22.4 | 2.1 | 24.5 |
| 12 | $Th_{0.11}Fe_{0.54}\ PO_x$ | 19.8 | 2.7 | 22.5 |
| 13 | $K_{0.4}Cr_{0.57}\ PO_x$ | 15.8 | 1.0 | 16.8 |
| 14 | $K_{0.4}Th_{0.48}Co_{0.48}\ PO_x$ | 7.5 | 0.0 | 7.5 |
| 15 | $K_{0.4}Th_{0.58}Ag_{0.58}\ PO_x$ | 53.3 | 1.6 | 54.9 |
| 16 | $K_{0.4}Th_{0.09}Fe_{0.45}Mo_{0.1}\ PO_x$ | 39.5 | 8.5 | 48.0 |

TABLE III
Reaction of Methyl Acetate with Methanol and Oxygen Over Vanadium-Antimony Catalysts

| Example | Catalyst | Per Pass Conversion % Methyl Acrylate | Acrylic Acid | Total |
|---|---|---|---|---|
| 17 | $VSb\ O_x$ | 35.4 | 9.6 | 45.0 |
| 18 | $VSb_5\ O_x$ | 36.6 | 10.8 | 47.4 |
| 19 | $VSb_5\ O_x$ | 42.8 | 11.3 | 54.1 |
| 20 | $K_{0.4}VSb_5\ O_x$ | 39.3 | 5.5 | 44.8 |
| 21 | $K_{0.4}Cu_{0.5}VSb_5\ O_x$ | 20.3 | 6.0 | 26.3 |
| 22 | $K_{0.4}Ag_{0.5}VSb_5\ O_x$ | 33.5 | 7.7 | 41.2 |
| 23 | $K_{0.4}Fe_{0.5}VSb_5\ O_x$ | 33.3 | 6.5 | 39.8 |
| 24 | $K_{0.4}Mo_{0.5}VSb_5\ O_x$ | 32.5 | 8.5 | 41.0 |
| 25 | $U_{0.5}VSb_5\ O_x$ | 35.8 | 9.1 | 44.9 |

TABLE IV
Reaction of Methyl Propionate with Methanol and Oxygen Over Various Catalysts

| Catalyst | Contact Time Sec. | $O_2$ MeOH | Per Pass Conversion % Methyl Methacrylate | Methacrylic Acid | Total |
|---|---|---|---|---|---|
| $VSb\ O_x$ | 6 | 0.4 | 17.5 | 3.0 | 20.5 |
| $VSb_5\ O_x$ | 6 | 0.4 | 22.0 | 2.3 | 24.3 |
| $K_{0.4}VSb_5\ O_x$ | 10 | 0.8 | 23.3 | 2.5 | 25.8 |
| $K_{0.4}Fe_{0.5}Ce_{0.47}\ PO_x$ | 10 | 0.8 | 7.9 | 0.5 | 8.4 |

**Claims**

1. A process for the production of unsaturated acids and esters which comprises reacting in the vapor phase at a temperature of 200°C to 500°C a first reactant selected from saturated monocarboxylic acids, esters and anhydrides, a second reactant selected from primary and secondary alcohols and di-alkyl ethers, and oxygen, in the presence of an oxidation catalyst, said catalyst having the empirical formula:

$$A_aB_bC_cPO_x$$

where
A = alkali metal, alkaline earth metal, Tl, or mixtures thereof
B = Fe, Co, Ni, Cr, Mn, Cu, Ag, Pt, Pd, Rh, Ru, or mixtures thereof
C = Sc, Y, La, rare earth metals, Th, U, Nb, Si, Ge, Ta, Mo, W, Bi, Sb, Te or mixtures thereof
where
a = 0—1.5
b = 0.01—3.0
c = 0—2.0
x = the number of oxygens required to satisfy the valence requirements of the other elements present.

2. A process as claimed in claim 1 wherein the first reactant has 2—14 carbon atoms.

3. A process as claimed in claim 2 characterised in that the first reactant is a saturated monocarboxylic acid, or an ester thereof, preferably acetic acid or propionic acid, or ester of such acid.

4. A process as claimed in any of claims 1 to 3 characterised in that the second reactant is an alcohol selected from ethanol, n-propanol, n-butanol, isobutanol and methanol.

5. A process as claimed in claim 4 characterised in that the alcohol is methanol and the first reactant is methylacetate or methylpropionate.

6. A process as claimed in any of claims 1 to 3 characterised in that the second reactant is dimethyl ether.

7. A process as claimed in claim 6 characterised in that the first reactant is methyl acetate or methyl propionate.

8. A process as claimed in any of claims 1 to 7 characterised in that the alkali metal is potassium or cesium.

9. A process as claimed in any of claims 1 to 8 characterised in that member B of the oxidation catalyst is copper, chromium, iron or silver.

10. A process as claimed in any of claims 1 to 9 characterised in that member C of the oxidation catalyst is thorium or cerium.

11. A process as claimed in any of claims 1 to 9 characterised in that member C of the oxidation catalyst is one or more elements selected from cerium, lanthanum, thorium, molybdenum, and tungsten.

12. A process as claimed in claim 1 characterised in that the oxidation catalyst has the empirical formula

$$K_{0.2-0.6}Ce_{0.45-0.65}Cu_{0.45-0.65}PO_x$$

or the empirical formula

$$K_{0.2-0.6}Ce_{0.05-0.2}Fe_{0.4-0.6}Mo_{0.05-0.8}PO_x.$$

13. A process for the production of unsaturated acids and esters which comprises reacting in the vapor phase at a temperature of 200°C to 500°C a first reactant selected from saturated monocarboxylic acids, esters and anhydrides, a second reactant selected from primary and secondary alcohols and di-alkyl ethers, and oxygen, in the presence of an oxidation catalyst, said catalyst having the empirical formula:

$$A_aB_bC_cVSb_mO_x$$

where

A = alkali metal, alkaline earth metal, Tl, La, rare earth metal, Th, or mixtures thereof;
B = Cu, Ag, Fe, Co, Ni, Mn, Cr, Nb, Ta, Ti, P, As, Sn, B, U, or mixtures thereof;
C = Mo, W, Te, Bi, or mixtures thereof;
a = 0—1
b = 0—1
c = 0—1
m = 0.5—40, preferably 3—15;
x = the number of oxygens required to satisfy the valence requirements of the other elements present.

14. A process as claimed in claim 13 characterised in that B of the catalyst is iron or copper.

15. A process as claimed in claim 13 characterised in that the oxidation catalyst has the empirical formula $VSb_5O_x$ or the empirical formula $VSb_{10}O_x$.

16. A process as claimed in claim 13 or claim 14 characterised in that the alkali metal is potassium or cesium.

**Patentansprüche**

1. Verfahren zur Herstellung von ungesättigten Säuren und Estern durch Umsetzung eines ersten Reaktanten, ausgewählt aus gesättigten Monocarbonsäuren, Estern und Anhydriden, eines zweiten Reaktanten, ausgewählt aus primären und sekundären Alkoholen und Dialkylethern, und Sauerstoff in der Dampfphase bei einer Temperatur von 200°C bis 500°C in Gegenwart eines Oxidationskatalysators, welcher Katalysator die empirische Formel aufweist:

$$A_aB_bC_cPO_x$$

worin

A = Alkalimetall, Erdalkalimetall, Tl oder Mischungen derselben,
B = Fe, Co, Ni, Cr, Mn, Cu, Ag, Pt, Pd, Rh, Ru oder Mischungen derselben,
C = Sc, Y, La, Seltenerdmetall, Th, U, Nb, Si, Ge, Ta, Mo, W, Bi, Sb, Te oder Mischungen derselben,

worin

a = 0—1,5
b = 0,01—3,0
c = 0—2,0

x = die Zahl der Sauerstoffatome, welche zur Befriedigung der Valenzbedürfnisse der anderen gegenwärtigen Elemente benötigt werden.

2. Verfahren nach Anspruch 1, worin der erste Reaktant 2—14 Kohlenstoffatome aufweist.

3. Verfahren nach Anspruch 2, dadurch gekennzeichnet, daß der erste Reaktant eine gesättigte Monocarbonsäure oder ein Ester davon ist, vorzugsweise Essigsäure oder Propionsäure oder ein Ester dieser Säure.

4. Verfahren nach einem der Ansprüche 1 bis 3, dadurch gekennzeichnet, daß der zweite Reaktant ein Alkohol ausgewählt aus Ethanol, n-Propanol, n-Butanol, Isobutanol und Methanol ist.

5. Verfahren nach Anspruch 4, dadurch gekennzeichnet, daß der Alkohol Methanol und der erste Reaktant Methylacetat oder Methylpropionat ist.

6. Verfahren nach einem der Ansprüche 1 bis 3, dadurch gekennzeichnet, daß der zweite Reaktant Dimethylether ist.

7. Verfahren nach Anspruch 6, dadurch gekennzeichnet, daß der erste Reaktant Methylacetat oder Methylpropionat ist.

8. Verfahren nach einem der Ansprüche 1 bis 7, dadurch gekennzeichnet, daß das Alkalimetall Kalium oder Cäsium ist.

9. Verfahren nach einem der Ansprüche 1 bis 8, dadurch gekennzeichnet, daß die Komponente B des Oxidationskatalysators Kupfer, Chrom, Eisen oder Silber ist.

10. Verfahren nach einem der Ansprüche 1 bis 9, dadurch gekennzeichnet, daß die Komponente C des Oxzidationskatalysators Thorium oder Cer ist.

11. Verfahren nach Anspruch 1 bis 9, dadurch gekennzeichnet, daß die Komponente C des Oxidationskatalysators aus einem oder mehreren Elementen ausgewählt aus Cer, Lanthan, Thorium, Molybdän und Wolfram besteht.

12. Verfahren nach Anspruch 1, dadurch gekennzeichnet, daß der Oxidationskatalysator die empirische Formel

$$K_{0,2-0,6}Ce_{0,45-0,65}Cu_{0,45-0,65}PO_x$$

oder die empirische Formel

$$K_{0,2-0,6}Ce_{0,05-0,2}Fe_{0,4-0,6}Mo_{0,05-0,8}PO_x.$$

aufweist.

13. Verfahren zur Herstellung von ungesättigten Säuren und Estern durch Umsetzung eines ersten Reaktanten, ausgewählt aus gesättigten Monocarbonsäuren, Estern und Anhydriden, eines zweiten Reaktanten, ausgewählt aus primären und sekundären Alkholen und Dialkylethern, und Sauerstoff in der Gasphase bei einer Temperatur von 200°C bis 500°C in der Gegenwart eines Oxidationskatylsators, welcher Katalysator die empirische Formel aufweist:

$$A_aB_bC_cVSb_mO_x$$

worin

A = Alkalimetall, Erdalkalimetall, Tl, La, Seltenerdmetall, Th oder Mischungen derselben;

B = Cu, Ag, Fe, Co, Ni, Mn, Cr, Nb, Ta, Ti, P, As, Sn, B, U oder Mischungen derselben;

C = Mo, W, Te, Bi oder Mischungen derselben;

a = 0—1

b = 0—1

c = 0—1

m = 0,5—40, vorzugsweise 3—15;

x = die Zahl der Sauerstoffatome, welche zur Befriedigung der Valenzbedürfnisse der anderen vorhandenen Elemente benötigt werden.

14. Verfahren nach Anspruch 13, dadurch gekennzeichnet, daß B des Katalysators Eisen oder Kupfer ist.

15. Verfahren nach Anspruch 13, dadurch gekennzeichnet, daß der Oxidationskatalysator die empirische Formel $VSb_5O_x$ oder die empirische Formel $VSb_{10}O_x$ aufweist.

16. Verfahren nach Anspruch 13 oder Anspruch 14, dadurch gekennzeichnet, daß das Alkalimetall Kalium oder Cäsium ist.

**Revendications**

1. Procédé de production d'acides et d'esters insaturés, qui consiste à faire réagir en phase vapeur, à une température de 200°C à 500°C, un premier réactif choisi parmi les acides, esters et anhydrides monocarboxyliques saturés, un second réactif choisi parmi les alcools primaires et secondaires et les éthers dialkyliques et l'oxygène, en présence d'un catalyseur d'oxydation, ce catalyseur répondant à la formule empirique:

$$A_aB_bC_cPO_x$$

dans laquelle

A = métal alcalin, métal alcalino-terreux, Tl ou leurs mélanges

B = Fe, Co, Ni, Cr, Mn, Cu, Ag, Pt, Pd, Rh, Ru ou leurs mélanges

C = Sc, Y, La, des métaux des terres rares, Th, U, Nb, Si, Ge, Ta, Mo, W, Bi, Sb, Te ou leurs mélanges

ou

a = 0—1,5

b = 0,01—3,0

c = 0—2,0

x = nombre d'oxygènes nécessaires pour satisfaire aux exigences de valence des autres éléments présents.

2. Procédé selon la revendication 1, dans lequel le premier réactif a 2 à 14 atomes de carbone.

3. Procédé selon la revendication 2, caractérisé par le fait que le premier réactif est un acide monocarboxylique saturé, ou un de ses esters, de préférence l'acide acétique ou l'acide propionique, ou un ester d'un tel acide.

4. Procédé selon l'une quelconque des revendications 1 à 3, caractérisé par le fait que le second réactif est un alcool choisi parmi l'éthanol, le n-propanol, le n-butanol, l'isobutanol et le méthanol.

5. Procédé selon la revendication 4, caractérisé par le fait que l'alcool est le mèthanol et le premier réactif est l'acétate de méthyle ou le propionate de méthyle.

6. Procédé selon l'une quelconque des revendications 1 à 3, caractérisé par le fait que le second réactif est l 'éther diméthylique.

7. Procédé selon la revendication 6, caractérisé par le fait que le premier réactif est l'acétate de méthyle ou le propionate de méthyle.

8. Procédé selon l'une quelconque des revendications 1 à 7, caractérisé par le fait que le métal alcalin est le potassium ou le césium.

9. Procédé selon l'une quelconque des revendications 1 à 8, caractérisé par le fait que l'élément B du catalyseur d'oxydation est le cuivre, le chrome, le fer ou l'argent.

10. Procédé selon l'une quelconque des revendications 1 à 9, caractérisé par le fait que l'élément C du catalyseur d'oxydation est le thorium ou le cérium.

11. Procédé selon l'une quelconque des revendications 1 à 9, caractérisé par le fait que l'élément C du catalyseur d'oxydation est un ou plusieurs éléments choisis parmi le cérium, le lanthane, le thorium, le molybdène et le tungstène.

12. Procédé selon la revendication 1, caractérisé par le fait que le catalyseur d'oxydation répond à la formule empirique

$$K_{0,2-0,6}Ce_{0,45-0,65}Cu_{0,45-0,65}PO_x$$

ou la formule empirique

$$K_{0,2-0,6}Ce_{0,05-0,2}Fe_{0,4-0,6}Mo_{0,05-0,8}PO_x.$$

13. Procédé pour la production d'acides et d'esters insaturés, qui consiste à faire réagir en phase vapeur, à une température de 200°C à 500°C, un premier réactif choisi parmi les acides, esters et anhydrides monocarboxyliques saturés, un second réactif choisi parmi les alcools primaires et secondaires et les éthers dialkyliques, et l'oxygène, en présence d'une catalyseur d'oxydation, ce catalyseur répondant à la formule empirique:

$$A_aB_bC_cVSb_mO_x$$

dans laquelle

A = métal alcalin, métal alcalino-terreux, Tl, La, métal des terres rares, Th ou des mélanges de ceux-ci;

B = Cu, Ag, Fe, Co, Ni, Mn, Cr, Nb, Ta, Ti, P, As, Sn, B, U, ou leurs mélanges;

C = Mo, W, Te, Bi ou leurs mélanges;

a = 0—1

b = 0—1

c = 0—1

m = 0,5—40, de préférence 3—15;

x = nombre d'oxygènes nécessaires pour satisfaire aux exigences de valence des autres éléments présents.

14. Procédé selon la revendication 13, caractérisé par le fait que B du catalyseur est le fer ou le cuivre.

15. Procédé selon la revendication 13, caractérisé par le fait que le catalyseur d'oxydation répond à la formule empirique $VSb_5O_x$ ou à la formule empirique $VSb_{10}O_x$.

16. Procédé selon la revendication 13 ou la revendication 14, caractérisé par le fait que le métal alcalin est le potassium ou le césium.